# EUROPEAN PATENT SPECIFICATION

(11) **EP 2 902 034 B1**
(45) Date of publication and mention of the grant of the patent: **09.01.2019**
(21) Application number: 12885310.8
(22) Date of filing: 28.09.2012
(51) Int. Cl.: A61K 36/77, A61P 13/12, A61P 29/00, A61K 131/00

(54) **USE OF LONGAN SEED ALCOHOL EXTRACT**
VERWENDUNG VON ALKOHOLEXTRAKT AUS LONGANSAMEN
UTILISATION D'EXTRAIT ALCOOLIQUE DE GRAINE DE LONGANE

(43) Date of publication of application: 05.08.2015
(73) Proprietor: JOBEN BIO-MEDICAL CO., LTD., Changzhi, Pingtung 90846 (TW)
(72) Inventor: TSENG, Huang-Chung, Changzhi Township Pingtung County 90846 Taiwan (TW); HSIAO, Yi-Ting, Changzhi Township Pingtung County 90846 Taiwan (TW); ZHAN, You-Zhong, Changzhi Township Pingtung County 90846 Taiwan (TW); YEN, Chun-Yi, Changzhi Township Pingtung County 90846 Taiwan (TW); LIN, Jian-Sheng, Changzhi Township Pingtung County 90846 Taiwan (TW)
(74) Representative: 2K Patentanwälte Blasberg Kewitz & Reichel
(86) International application number: PCT/CN2012/082275
(87) International publication number: WO 2014/047865

(56) References cited:
- WO-A1-2011/003236
- ABDURRAUF YÜCE ET AL: "Amelioration of Cyclosporine A-Induced Renal, Hepatic and Cardiac Damages by Ellagic Acid in Rats*", BASIC & CLINICAL PHARMACOLOGY & TOXICOLOGY, vol. 103, no. 2, 1 August 2008 (2008-08-01), pages 186-191, XP055268905, COPENHAGEN, DK ISSN: 1742-7835, DOI: 10.1111/j.1742-7843.2008.00284.x
- Suoyi Huang ET AL: "The Total Flavanone of Longan Seed Extraction and the Identification by Ethanol", LISHIZHEN MEDICINE AND MATERIA MEDICA RESEARCH, Vol. 18, No. 4, 1 January 2007 (2007-01-01), pages 1021-1023, XP055268796, Retrieved from the Internet: URL:http://www.taozhy.com/baokan/2778.thtm l [retrieved on 2016-04-26]
- MENG-CHIEH HSIEH ET AL: "Antioxidative Activity and Active Components of Longan (Dimocarpus longan Lour.) Flower Extracts", JOURNAL OF AGRICULTURAL AND FOOD CHEMISTRY, vol. 56, no. 16, 1 August 2008 (2008-08-01), pages 7010-7016, XP055268900, US ISSN: 0021-8561, DOI: 10.1021/jf801155j
- NUCHANART RANGKADILOK ET AL: "Identification and Quantification of Polyphenolic Compounds in Longan ( Euphoria longana Lam.) Fruit", JOURNAL OF AGRICULTURAL AND FOOD CHEMISTRY, vol. 53, no. 5, 8 February 2005 (2005-02-08), pages 1387-1392, XP055268902, US ISSN: 0021-8561, DOI: 10.1021/jf0403484
- SOONG Y Y ET AL: "Quantification of gallic acid and ellagic acid from longan (Dimocarpus longan Lour.) seed and mango (Mangifera indica L.) kernel and their effects on antioxidant activity", FOOD CHEMISTRY, ELSEVIER LTD, NL, vol. 97, no. 3, 1 August 2006 (2006-08-01) , pages 524-530, XP027989301, ISSN: 0308-8146 [retrieved on 2006-08-01]
- S. Q. ZHENG ET AL: "Preliminary observations on the antifatigue effects of longan ( Dimocarpus longan Lour.) seed polysaccharides", PHYTOTHERAPY RESEARCH., 1 January 2010 (2010-01-01), pages n/a-n/a, XP055269041, GB ISSN: 0951-418X, DOI: 10.1002/ptr.2963
- HOU CHIEN-WEI ET AL: "Longan seed extract reduces hyperuricemia via modulating urate transporters and suppressing xanthine oxidase activity.", THE AMERICAN JOURNAL OF CHINESE MEDICINE 2012, vol. 40, no. 5, 1 January 2012 (2012-01-01), pages 979-991, XP009189826, ISSN: 0192-415X
- WANG, SHUXIA ET AL.: 'HPLC-ESI-MS Analysis of Phenolic Compounds in Different Solvent Fractions of Ethanol Extract of Longan Seeds and Their Antioxidant Activities' FOOD SCIENCE vol. 32, no. 12, June 2011, pages 196 - 203, XP055248183
- LI, XUEHUA ET AL.: 'Study on the Anti-Oxidation Effects of Longan Seeds Ethyl Acetate Fraction' LISHIZHEN MEDICINE AND MATERIA MEDICA RESEARCH vol. 19, no. 8, August 2008, pages 169 - 171, XP055248184
- LI, XUEHUA ET AL.: 'Studies on the Chemical Constituents from the Longan Seeds' ACTA MEDICINAE UNIVERSITATIS SCIENTIAE ET TECHNOLOGIAE HUAZHONG vol. 38, no. 4, August 2009, pages 524 - 526, XP055248650
- FENG, LI ET AL.: 'Progress in Plant Polyphenols and Their Physiological Functions' ACTA AGRICULTURAE JIANGXI vol. 19, no. 10, October 2007, pages 105 - 107, XP055248196
- JU, HAISONG ET AL. CHINESE PHARMACOLOGICAL BULLETIN vol. 5, no. 5, October 1989, pages 263 - 267, XP008178948

## Description

### BACKGROUND OF THE INVENTION

### FIELD OF THE INVENTION

The invention relates to use of an extract of longan seeds; more particularly, in the treatment of kidney tissue dysfunction.

### DESCRIPTION OF THE RELATED ART

Kidneys are located at both sides of the spine at the rear of abdominal cavity in human body, namely, in an included area where the last rib (the twelfth rib) and the spine are jointed. The basic unit of the kidney is nephron, each kidney includes about one million nephrons, and each nephron includes renal glomeruli and renal tubules.

Functions of the nephrons include: filtering wastes, water and electrolytes of the body, and producing urine, for example, removing excessive water; and removing uric acid, urea, creatinine and medicines; and controlling stabilization and balance of electrolytes such as sodium, potassium, calcium, phosphorus, and acids and bases, and maintaining stabilization of the in vivo environment. In addition to mechanism of producing urine, the kidneys can further excrete: (1) erythropoietin, if renal injuries leads to erythropoietin hyposecretion, anemia occurs; (2) D3 for activating vitamins, which is the most important ingredient for maintaining the balance of calcium and phosphorus in blood, so renal failure may lead to bone lesions; and (3) rennin and angiotensin, for adjusting blood pressure, and therefore many types of hypertension are related to kidney troubles.

Many factors are causes of kidney tissue dysfunctions: (1) congenital kidney diseases, such as polycystic kidney disease, and hereditary nephritis and urinary tact abnormalities; (2) glomerulopathy, such as primary glomerulonephritis and secondary glomerulonephritis, for example, diabetes mellitus, hypertension and lupus erythematosus; (3) tubular interstitial nephropathy, such as nephrolithiasis, renal tumor, and urethral or ureteral stricture; (4) vascular kidney diseases, such as vasculitis and hypertension nephrosclerosis; and (5) infection, such as nephritis caused by bacterial infections. In addition, due to aging, long-term drug abuse, family inheritance and high-salt diet, obesity, high cholesterol, smoking and alcohol are causes of kidney tissue dysfunctions. The clinical symptoms include changes in urine pattern such as hyperuresis (especially in night), hematuria, urine blister (urinary protein may be contained); and physical discomfort, such as eyelid edemas or face, hands and feet edema, high blood pressure, anemia, itchy skin, general tiredness, heart failure and pulmonary edema.

However, there are many causes of kidney tissue dysfunctions, there is no effective drugs that can effectively treat kidney tissue dysfunctions, for example, in treatment of secondary kidney tissue dysfunctions, primary focuses are treated to control the disease; in treatment of kidney tissue dysfunctions caused by infections, antibiotics need to be used; and in treatment of nephritis, not only common anti inflammatory agents cannot be used, but also the symptoms get more severe due to use of the common anti-inflammatory agents, and steroids or immunosuppressants are generally used, but serious side effects are serious are caused. Therefore, when the course of kidney tissue dysfunctions is continuously developed, treatment such as peritoneal dialysis even renal transplantation is required, which has great influence on the patient's living and life.

Longan is the fruit of the Family Sapindaceae and Genus Dimocarpus plant, and also called as Guiyuan. Longan can be used in herbal medicine. The pulp has a sweet taste and is warm in nature, and has the effect of invigoration; the stone has an astringent taste and is neutral in nature, and has the effect of convergence and stopping bleeding; the leave has a light taste and is neutral in nature, and has the effect of resolving superficies syndrome. Longan has a variety functions such as invigoration and benefiting qi, helping building blood, invigorating heart and spleen, nourishing blood and tranquilizing mind, moisturizing skin and beautification, and can be used to treat anemia, palpitations, insomnia, amnesia, neurasthenia and post-illness and postpartum weakness. Longan seed is used in trauma treatment since ancient times, and as recorded in ancient book A Collection of Chinese Herbal Medicines, longan stone can be used in the treatment of stomach pain, burn and scald wounds, bleeding, ulcer pain, trauma bleeding, scabies and eczema. When being used for trauma treatment by ancients, longan seed has good hemostatic effect, pain-relieving effect and function of regeneration of tissues, and is known as "Jindao Dusheng San". In recent years, studies related to the health care effects of longan, especially studies in the antioxidant capacity, effective ingredients and whitening capability are gradually increasing. Most studies have found that, the flower and fruit of longan have high-level antioxidants, such as gallic acid, corilagin) and ellagic acid. However, studies on longan seed are relatively few. Generally, after longan pulp is obtained, longan seed is abandoned.

Although many uses of longan have been reported, different uses of the extract of longan seeds are still to be developed.

### SUMMARY OF THE INVENTION

The invention is defined in the claims. The present invention provides an alcohol extract of longan seeds for use in the treatment of kidney tissue dysfunction.

Further described is a method for treating kidney tissue dysfunction in a subject, which comprises administering to said subject an effective amount of an alcohol extract of longan seeds and optionally a pharmaceutically acceptable carrier or excipient.

The disclosure describes a pharmaceutical composition for treating kidney tissue dysfunction comprising an alcohol extract of longan seeds and optionally a pharmaceutically acceptable carrier or excipient.

A process for preparing an alcohol extract of longan seeds comprises:
(a) providing longan seeds;
(b) cutting the longan seeds into small pieces; and
(c) extracting the small pieces in step (b) with the alcohol to obtain an extract.

### BRIEF DESCRIPTION OF THE DRAWINGS

FIG. 1 illustrates the GC-MS spectrogram of the alcohol extract of longan seeds according to the invention.
FIG. 2 illustrates the flash chromatography spectrogram of the alcohol extract of longan seeds according to the invention.
FIG. 3 illustrates the HPLC spectrogram of the alcohol extract of longan seeds according to the invention.
FIG. 4 illustrates the weight-time diagram of the experimental animals in the 5/6 nephrectomized rat model. The solvent for the extract includes 5% DMSO and 10% chlorophyll. The composition is given orally. The data are expressed as mean (n = 3 per group).
FIG. 5 illustrates the blood urea nitrogen in serum-time diagram of the experimental animals in the 5/6 nephrectomized rat model. The solvent for the extract includes 5% DMSO and 10% chlorophyll. The data are expressed as mean (n = 3 per group).
FIG. 6 illustrates the blood creatinine-time diagram of the experimental animals in the 5/6 nephrectomized rat model. The data are expressed as mean (n = 3 per group).
FIG. 7 illustrates the urine protein-creatinine ratio-time diagram of the experimental animals in the 5/6 nephrectomized rat model. The data are expressed as mean (n = 3 per group).
FIG. 8 illustrates the kidney tissue pathological slices of the experimental animals in the 5/6 nephrectomized rat model.

### DETAILED DESCRIPTION OF THE INVENTION

The present invention provides an alcohol extract of longan seeds for use in the treatment of kidney tissue dysfunction. The pharmaceutical composition comprising the alcohol extract of longan seeds is used for treating kidney tissue dysfunction, and the pharmaceutical composition comprises an alcohol extract of longan seeds and optionally a pharmaceutically acceptable carrier or excipient.

The present invention can be more readily understood by reference to the following detailed description of various embodiments of the invention, the examples, and the chemical drawings and tables with their relevant descriptions. It is to be understood that unless otherwise specifically indicated by the claims, the invention is not limited to specific preparation methods, carriers or formulations, or to particular modes of formulating the extracts of the invention into products or compositions intended for topical, oral or parenteral administration, because as one of ordinary skill in the relevant arts is well aware, such things can, of course, vary. It is also to be understood that the terminology used herein is for the purpose of describing particular embodiments only and is not intended to be limiting.

As utilized in accordance with the present disclosure, the following terms, unless otherwise indicated, shall be understood to have the following meaning:

Often, ranges are expressed herein as from "about" one particular value and/or to "about" another particular value. When such a range is expressed, an embodiment includes the range from the one particular value and/or to the other particular value. Similarly, when values are expressed as approximations, by use of the word "about," it will be understood that the particular value forms another embodiment. It will be further understood that the endpoints of each of the ranges are significant both in relation to and independently of the other endpoint. As used herein the term "about" refers to ± 10%.

"Optional" or "optionally" means that the subsequently described event or circumstance may or may not occur, and that the description includes instances where said event or circumstance occurs and instances where it does not. For example, the phrase "optionally comprising an agent" means that the agent may or may not exist.

It must be noted that, as used in the specification and the appended claims, the singular forms "a," "an" and "the" include plural referents unless the context clearly dictates otherwise. Thus, unless otherwise required by context, singular terms shall include the plural and plural terms shall include the singular.

The term "subject" as used herein denotes any animal, preferably a mammal, and more preferably a human. The examples of subjects include humans, non-human primates, rodents, guinea pigs, rabbits, sheep, pigs, goats, cows, horses, dogs and cats.

The term "effective amount" of an active ingredient as provided herein means a sufficient amount of the ingredient to provide the desired regulation of a desired function, such as gene expression, protein function, or the induction of a particular type of response. As will be pointed out below, the exact amount required will vary from subject to subject, depending on the disease state, physical conditions, age, sex, species and weight of the subject, the specific identity and formulation of the composition, etc. Dosage regimens may be adjusted to induce the optimum therapeutic response. For example, several divided doses may be administered daily or the dose may be proportionally reduced as indicated by the exigencies of the therapeutic situation. Thus, it is not possible to specify an exact "effective amount." However, an appropriate effective amount can be determined by one of ordinary skill in the art using only routine experimentation.

The term "treating" or "treatment" as used herein denotes reversing, alleviating, inhibiting the progress of, or improving the disorder or condition to which such term applies, or one or more symptoms of such disorder or condition.

The term "carrier" or "excipient" as used herein refers to any substance, not itself a therapeutic agent, used as a carrier and/or diluents and/or adjuvant, or vehicle for delivery of a therapeutic agent to a subject or added to a formulation to improve its handling or storage properties or to permit or facilitate formation of a dose unit of the composition into a discrete article such as a capsule or tablet suitable for oral administration. Suitable carriers or excipients are well known to persons of ordinary skill in the art of manufacturing pharmaceutical formulations or food products. Carriers or excipients can include, by way of illustration and not limitation, buffers, diluents, disintegrants, binding agents, adhesives, wetting agents, polymers, lubricants, glidants, substances added to mask or counteract a disagreeable taste or odor, flavors, dyes, fragrances, and substances added to improve appearance of the composition. Acceptable carriers or excipients include citrate buffer, phosphate buffer, acetate buffer, bicarbonate buffer, stearic acid, magnesium stearate, magnesium oxide, sodium and calcium salts of phosphoric and sulfuric acids, magnesium carbonate, talc, gelatin, acacia gum, sodium alginate, pectin, dextrin, mannitol, sorbitol, lactose, sucrose, starches, gelatin, cellulosic materials (such as cellulose esters of alkanoic acids and cellulose alkyl esters), low melting wax cocoa butter, amino acids, urea, alcohols, ascorbic acid, phospholipids, proteins (for example, serum albumin), ethylenediamine tetraacetic acid (EDTA), dimethyl sulfoxide (DMSO), sodium chloride or other salts, liposomes, mannitol, sorbitol, glycerol or powder, polymers (such as polyvinyl-pyrrolidone, polyvinyl alcohol, and polyethylene glycols), and other pharmaceutically acceptable materials. The carrier should not destroy the pharmacological activity of the therapeutic agent and should be non-toxic when administered in doses sufficient to deliver a therapeutic amount of the agent.

Preferably, the alcohol extract of longan seeds is comprised in a composition. The composition of the invention is preferably a food composition or a pharmaceutical composition.

The alcohol extract of longan seeds can be added to a conventional food composition (i.e. the edible food or drink or precursors thereof) in the manufacturing process of the food composition. Almost all food compositions can be supplemented with the alcohol extract of longan seeds of the invention. The food compositions that can be supplemented with the alcohol extract of longan seeds of the invention include, but are not limited to, candies, baked goods, ice creams, dairy products, sweet and flavor snacks, snack bars, meal replacement products, fast foods, soups, pastas, noodles, canned foods, frozen foods, dried foods, refrigerated foods, oils and fats, baby foods, or soft foods painted on breads, or mixtures thereof.

The pharmaceutical composition is preferably administered topically or systemically by any method known in the art, including, but not limited to, intramuscular, intradermal, intravenous, subcutaneous, intraperitoneal, intranasal, oral, mucosal or external routes. The appropriate route, formulation and administration schedule can be determined by those skilled in the art. The pharmaceutical composition can be formulated in various ways, according to the corresponding route of administration, such as a liquid solution, a suspension, an emulsion, a syrup, a tablet, a pill, a capsule, a sustained release formulation, a powder, a granule, an ampoule, an injection, an infusion, a kit, an ointment, a lotion, a liniment, a cream or a combination thereof. If necessary, it may be sterilized or mixed with any pharmaceutically acceptable carrier or excipient, many of which are known to one of ordinary skill in the art; see paragraph [0029] for example.

As used herein, the term "alcohol extract of longan seeds" refers to an extract obtained by extracting longan seeds with an alcohol solution. The manner of extracting seeds with a solution is well-known to artisans skilled in this field. In one preferred embodiment of the invention, the longan seeds are soaked in the alcohol solution for extraction; more preferably, the longan seeds are soaked in the alcohol solution and subjected to ultrasonic vibration extraction.

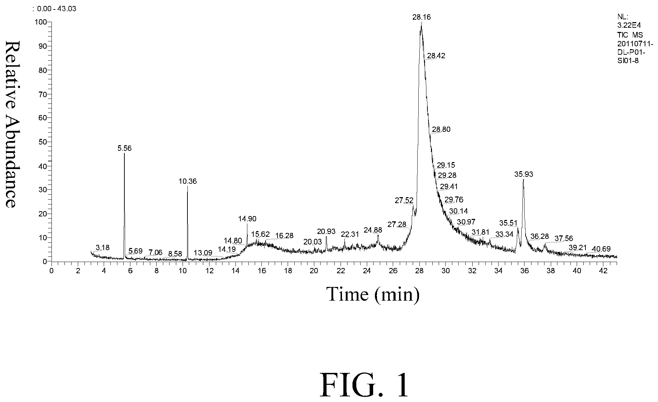

In one preferred embodiment of the invention, the alcohol extract of longan seeds is subjected to a Gas Chromatography-Mass Spectrophotometry (GC-MS) assay. The gas chromatography is conducted with Trace GC Ultra, thermo; and the mass spectrophotometry is conducted with ITQ 900, thermo; the column is Varian® VF-5ms 30 m × 0.25 mm (I.D. 0.25 µm). The temperature program is 150°C for 5 min; heating to 190°C at a rate of 5°C/min for 20 min. As shown in FIG. 1, the spectrogram obtained comprises peaks at retention time of about 5.56 min, about 10.36 min, 14.90 min, about 27.52 min, about 28.16 min, about 35.51 min, about 35.93 min, and about 37.56 min.

The longan seeds referred to in this invention are not particularly limited. Preferably, the longan belongs to Sapindaceae family and Dimocarpus genus, and also called as Guiyuan. More preferably, the longan is Dimocarpus longan Lour, Dimocarpus longan or Dimocarpus longan Fen Ke.

The longan seed is the nutlet part of longan fruit, and substantially does not include the shell and pulp part. The manner for obtaining the longan seed from the longan fruit is well-known to artisans skilled in this field.

In one preferred embodiment of the invention, the alcohol is selected from the group consisting of methanol, ethanol, n-propanol, isopropanol, n-butanol, iso-butanol, sec-butanol, tert-butanol, and ethyl acetate. More preferably, the alcohol is ethanol, n-butanol or ethyl acetate; most preferably, the alcohol is ethanol. The alcohol solution preferably about 20% to about 99.9% alcohol.

In one preferred embodiment of the invention, the alcohol extract of longan seeds is an ethanol extract of longan seeds.

In one preferred embodiment of the invention, the alcohol extract of longan seeds comprises an ethyl acetate fraction of the alcohol extract of longan seeds.

In another preferred embodiment of the invention, the alcohol extract of longan seeds comprises a water fraction obtained by extracting the alcohol extract of longan seeds with ethyl acetate.

In still another preferred embodiment of the invention, the alcohol extract of longan seeds comprises an n-butanol sub-fraction of the water fraction obtained by extracting the alcohol extract of longan seeds with ethyl acetate.

In still another preferred embodiment of the invention, the alcohol extract of longan seeds comprises a water sub-fraction obtained by extracting the water fraction (obtained by extracting the alcohol extract of longan seeds with ethyl acetate) with n-butanol.

In one more preferred embodiment of the invention, the alcohol extract of longan seeds comprises a fraction obtained by flash column chromatography; the column is 40g Silica; the flow rate is 18 mL/min; the mobile phase uses Solution A of ethyl acetate and Solution B of methanol; the gradient elution program is 100% Solution A and 0% Solution B at about 0 minute to about 30 minutes; 80% Solution A and 20% Solution B at about 31 minutes to about 45 minutes; 50% Solution A and 50% Solution B at about 46 minutes to about 65 minutes; 0% Solution A and 100% Solution B at about 66 minutes to about 85 minutes and at about 86 minutes to about 100 minutes. Still more preferably, the alcohol extract of longan seeds comprises a fraction obtained by flash column chromatography with the gradient elution program of 100% Solution A and 0% Solution B at about 0 minute to about 30 minutes.

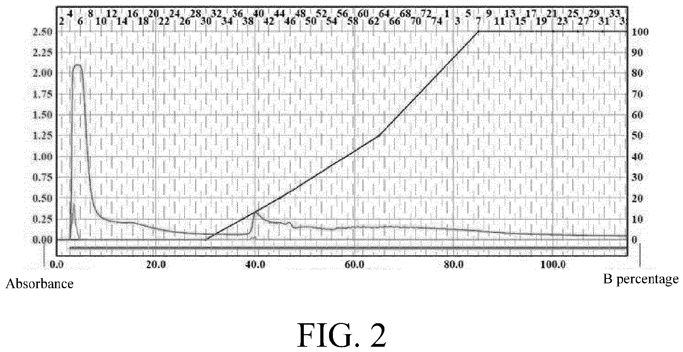

In one embodiment of the invention, the results of the ethyl acetate fraction of the alcohol extract of longan seeds isolated by flash column chromatography are shown in FIG. 2, and wherein DL-P01-SI01: 765.3 mg; DL-P01-SI02: 100.4 mg; DL-P01-SI03: 37.0 mg; DL-P01-SI04: 21.7 mg; DL-P01-SI05: 140.1 mg; DL-P01-SI06: 57.3 mg; DL-P01-SI07: 28.4 mg; DL-P01-SI08: 17.0 mg; DL-P01-SI09: 6.4 mg; DL-P01-SI10: 7.7 mg.

A process for preparing an alcohol extract of longan seeds comprises:
(a) providing longan seeds;
(b) cutting the longan seeds into small pieces; and
(c) extracting the small pieces in step (b) with the alcohol to obtain an extract.

According to the process, prior to step (b), the longan seeds are preferably dried.

Preferably, step (b) further comprises blending the small pieces into powder. The manner of cutting and/or blending is well-known to artisans skilled in this field.

The ratio (w/v) of the longan seeds and the alcohol solution is not specifically restricted, and can be about 1:1 to about 1:10; preferably about 1:3 to about 1:8; and most preferably about 1:5.

The extracting the small pieces in step (b) with the alcohol is with heat. Preferably, the temperature for the extracting in step (c) is about 30°C to about 90°C.

Preferably, step (c) further comprises:
(c1) extracting the small pieces in step (b) with an ethanol solution to obtain a rude extract;
(c2) lyophilizing the rude extract in step (c1); and
(c3) extracting a product of lyophilizing in step (c2) with ethyl acetate to obtain an ethyl acetate fraction and a water fraction.

Preferably, the rude extract is filtered and condensed prior to lyophilization.

In one more preferred embodiment of the disclosure, step (c) further comprises
(c4) extracting the water fraction with n-butanol to obtain an n-butanol sub-fraction and a water sub-fraction.

In one preferred embodiment of the disclosure, the preparations of the ethyl acetate fraction, water fraction, n-butanol subfraction or water sub-fraction of the alcohol extract of longan seeds are for further extracting the alcohol extract of longan seeds.

In one preferred embodiment of the disclosure, the process further comprises extracting the ethyl acetate fraction by flash column chromatography; wherein the column is 40g Silica; the flow rate is 18 mL/min; the mobile phase uses Solution A of ethyl acetate and Solution B of methanol; the gradient elution program is 100% Solution A and 0% Solution B at about 0 minute to about 30 minutes; 80% Solution A and 20% Solution B at about 31 minutes to about 45 minutes; 50% Solution A and 50% Solution B at about 46 minutes to about 65 minutes; 0% Solution A and 100% Solution B at about 66 minutes to about 85 minutes and at about 86 minutes to about 100 minutes.

Preferably, the process further comprises (d) obtaining a liquid fraction from the extract, and a solid fraction is removed. The manner of removing the solid fraction to obtain the liquid fraction is well known to artisans skilled in this field.

The kidney tissue dysfunction according to the present invention refers to a kidney tissue dysfunction caused by any causes, and preferably, the kidney tissue dysfunction is kidney tissue dysfunction caused by renal tissue necrosis, hypertension, immune injury, diabetes mellitus, systemic lupus erythematosus, aging, long-term drug abuse, family inheritance, high-salt diet, obesity, high cholesterol, smoking, alcohol or nephrectomy.

Detection of whether the kidney tissue function is normal can be diagnosed through the following tests: (1) detection of blood urea nitrogen (BUN) and serum creatinine (Cr) in blood, because kidney tissue dysfunctions cause increase of the two values; (2) detection of index of urinary protein in urine and blood urine, to calculate the glomerular filtration rate, so as to know the kidney function; (3) X-ray examination: the kidney appearance is inspected by X-ray examination, and at the same time, whether a kidney stone exists and the relative position of the kidney stone are detected; and (4) ultrasonic inspection: to know the size of the kidney, whether a kidney stone and tumor exist.

The kidney tissue dysfunction refers to that it is proved by diagnosis and inspection that abnormity is found in the kidney structure (histophysiological or imaging examination) or the kidney function (blood or urine inspection) or the glomerular filtration rate (GFR) is less than 60 cc/min/1.73m².

In an animal model of an embodiment of the present invention, in rats with 5/6 kidney resected, administration of the alcohol extract of longan seeds of the present invention can reduce the blood and/or urine urinary protein, reduce the blood urea nitrogen, reduce the urine protein-creatinine ratio, reduce the blood and/or urine creatine kinase, reduce the blood and/or urine lactate dehydrogenase, reduce the blood and/or urine lactic acid and alleviate segmental sclerosis.

In a preferred specific embodiment of the present invention, the kidney tissue dysfunction is selected from the group consisting of excessively high blood and/or urine urinary protein, excessively high blood urea nitrogen, excessively high urine protein-creatinine ratio, excessively high blood and/or urine creatine kinase, excessively high blood and/or urine lactate dehydrogenase, excessively high blood and/or urine lactic acid and segmental sclerosis.

The following examples are given for the purpose of illustration only and are not intended to limit the scope of the present invention.

### Example

### Alcohol extract of longan seeds

After shelling and removing the pulp from longan fruits, the longan seeds are obtained, and then longan seeds are smashed, and the smashed longan seeds are immersed in a 20% to 95% ethanol solution at a temperature of 30°C to 90°C, and the temperature is maintained at 30°C to 90°C for 1 to 3 hrs. The solution obtained through extraction is filtered, concentrated, and then subjected to low-temperature and low-pressure lyophilized, to obtain a rude extract (DL).

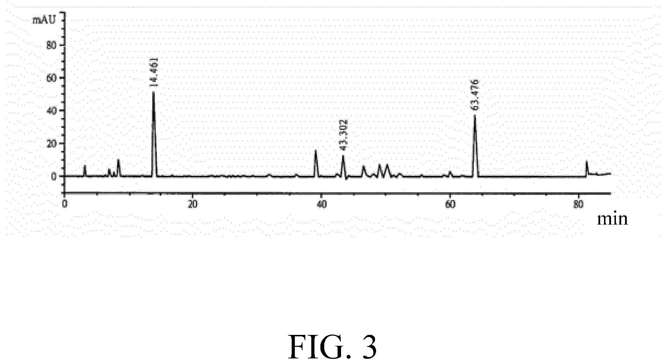

The rude extract is subjected to a high performance liquid chromatography assay as described in Table 1, and the result is shown in FIG.3.

**Table 1:**

| Apparatus | Agilent 1100 | | | | | | | |
|---|---|---|---|---|---|---|---|---|
| Chromatography column | Atlantis®T35 µm 4.6x10 mm , Waters | | | | | | | |
| Precolumn | Cosmosil 5C18-AR-II 4.6x10 mm , Nacalai Tesque | | | | | | | |
| Column temperature | 25°C | | | | | | | |
| Flow rate | 1.0 mL/min | | | | | | | |
| Detection wave length | UV 270 nm | | | | | | | |
| Injection volumn | 10 µL | | | | | | | |
| Time (min) | 0 | 5 | 15 | 30 | 45 | 60 | 75 | 85 |
| 0.1% H3PO4(%) | 98 | 97 | 97 | 87 | 86 | 81 | 79 | 0 |
| acetonitrile(%) | 2 | 3 | 3 | 13 | 14 | 19 | 21 | 100 |

The rude extract (DL) is further extracted with ethyl acetate to obtain an ethyl acetate fraction (DL-P01) and a water fraction. The water fraction is further extracted with n-butanol to obtain an n-butanol sub-fraction (DL-P02) and a water sub-fraction (DL-P03). After extracting 24.13 g of the rude extract, DL-P01: 1.44g (5.97%); DL-P02: 2.57g (10.65%); DL-P03: 19.11g (79.20%) are obtained.

The ethyl acetate fraction (DL-P01) is further subjected to the flash chromatography. About 1.1 g of DL-P01 is dissolved in ethyl acetate and methanol with the assistance of an ultrasonic cleaner, and then silica gel is added. After condensing to dry, the sample is placed into a empty sample tube. On the other hand, the normal phase column, 40g silica gel, for the flash chromatography is equipped to the apparatus. The column is saturated with the solvent and the sample is loaded. The program is listed below: the flow rate is 18 mL/min; the mobile phase uses Solution A of ethyl acetate and Solution B of methanol; the gradient elution program is 100% Solution A and 0% Solution B at about 0 minute to about 30 minutes; 80% Solution A and 20% Solution B at about 31 minutes to about 45 minutes; 50% Solution A and 50% Solution B at about 46 minutes to about 65 minutes; 0% Solution A and 100% Solution B at about 66 minutes to about 85 minutes and at about 86 minutes to about 100 minutes.

The results are shown in FIG. 2, and DL-P01-SI01: 765.3 mg; DL-P01-SI02: 100.4 mg; DL-P01-SI03: 37.0 mg; DL-P01-SI04: 21.7 mg; DL-P01-SI05: 140.1 mg; DL-P01-SI06: 57.3 mg; DL-P01-SI07: 28.4 mg; DL-P01-SI08: 17.0 mg; DL-P01-SI09: 6.4 mg; DL-P01-SI10: 7.7 mg are obtained. DL-P01-SI01 is the main product (69.86%) which is collected by flash column chromatography with the gradient elution program of 100% Solution A and 0% Solution B at about 0 minute to about 30 minutes.

About 0.6 mL of methanol is added in 6 mg of the above mentioned DL-P01-SI01 and further subjected to vibration, dissolve, and filtration, and then subjected to a Gas Chromatography-Mass Spectrophotometry (GC-MS) assay. The gas chromatography is conducted with Trace GC Ultra, thermo; and the mass spectrophotometry is conducted with ITQ 900, thermo; the column is Varian® VF-5ms 30 m × 0.25 mm (I.D. 0.25 µm). The temperature program is 150°C for 5 min; heating to 190°C at a rate of 5°C/min for 20 min. As shown in FIG. 1, the spectrogram obtained comprises peaks at retention time of about 5.56 min, about 10.36 min, 14.90 min, about 27.52 min, about 28.16 min, about 35.51 min, about 35.93 min, and about 37.56 min. Alcohol extract of longan seeds in treatment of kidney tissue dysfunction

In this example, a 5/6 nephrectomized rat model is used to perform in vivo pharmacological activity test and efficacy evaluation of kidney tissue dysfunction.

Sprague-Dawley white rates were adopted, the right kidney and 2/3 left kidney were resected through operation, and one week later, urinary protein in urine was detected to confirm the nephritis syndrome, and then the rats were grouped and subjected to administration tests. The nephritis animal were randomly grouped, including: a pseudo operation group (pseudo operation animal + solvent for formulating agent), a control group (nephritis animal + solvent for formulating agent), an experimental group (nephritis animal + DL-P01-SI01, referred to as J-TK for short), and the number of rats in each group was not less than three. Before administration, continuous observation was performed for eight weeks after drug treatment, blood was sampled at caudal vein and urine sample was collected by a metabolic cage once per week for tracking survey of physiological index data and biochemical analysis of urine and blood, urine was collected before sacrifice at the ninth week, and blood and tissue were collected after sacrifice for biochemical analysis.

The main items for physiological, biochemical analysis include: concentration detection of urinary protein, blood use a nitrogen, creatinine, creatine kinase, lactate dehydrogenase, lactic acid, and blood count measurement.

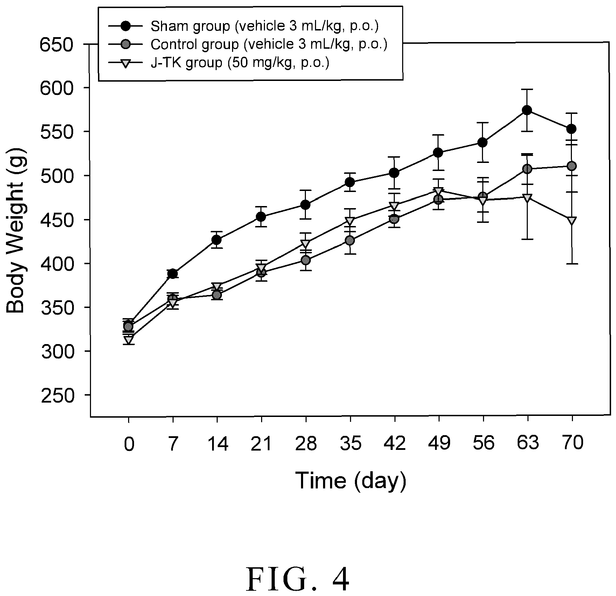

In this animal model experiment, it can be observed that the weights of the experimental animals are normally is slowly increased, as shown in FIG. 4.

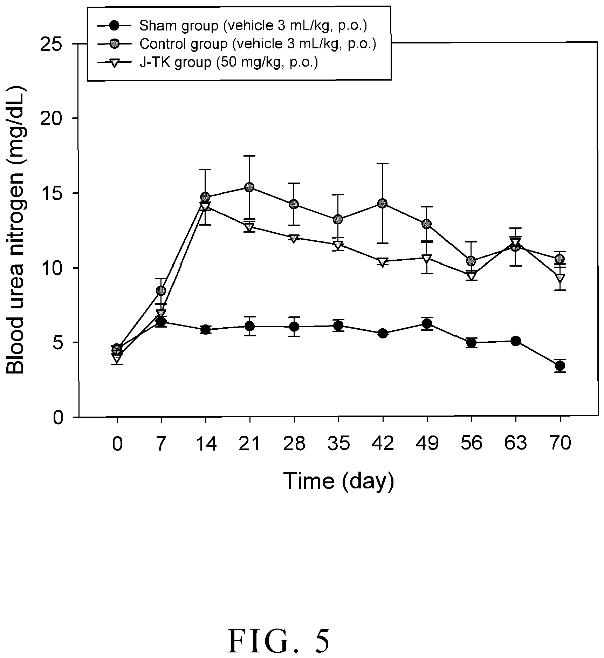

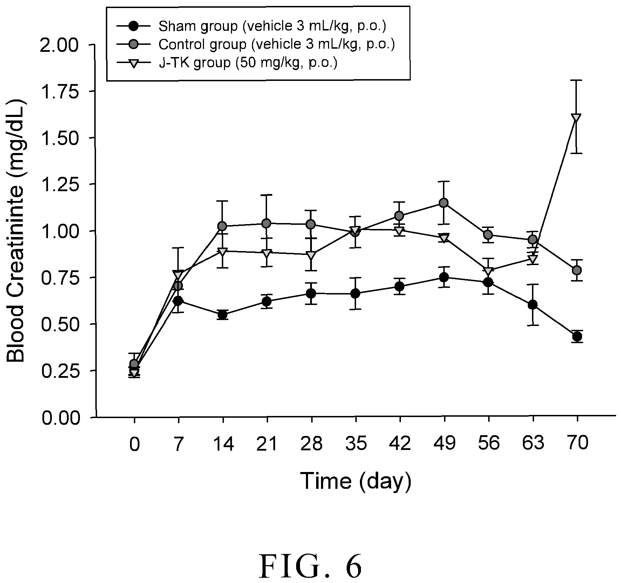

Biochemical indexes such as blood use a nitrogen, lactate dehydrogenase, lactic acid, creatinine in serum are detected. After the second operation, it can be found that blood use a nitrogen and creatinine in serum of operation animal are significantly increased. After administration and treatment, it is observed after one-week administration that DL-P01-SI01 (J-TK) can significantly decelerate the increase of blood use a nitrogen and creatinine in serum, and after four-week administration, the inhibition rate of J-TK on blood use a nitrogen in blood is up to 27.2%. The result indicates that in the animal model, the regulation effect on blood use a nitrogen is significant, as shown in FIG. 5 and FIG. 6.

It can be observed from the variation of lactic acid and lactate dehydrogenase that in this experiment, the physiology condition of the animal is not influenced by unexpected diseases, which is not shown in the figures.

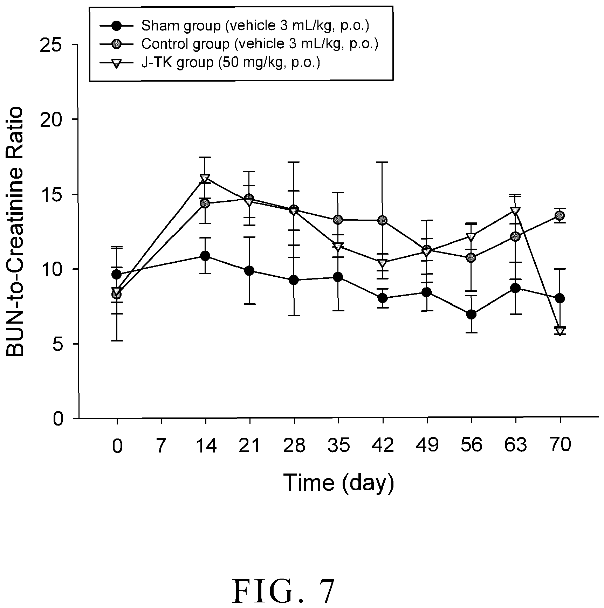

The analysis data of urine urinary protein and creatinine are presented by a ratio of single spot urinary protein concentration to urine creatinine concentration, and the results indicates that after nephrectomy, the urinary protein concentration/urine creatinine concentration in animal urine is significantly increased, compared with that of the pseudo operation group, and after three-week administration, the effect of decelerating the increase of the urinary protein concentration/urine creatinine concentration ratio is exhibited, as shown in FIG. 7.

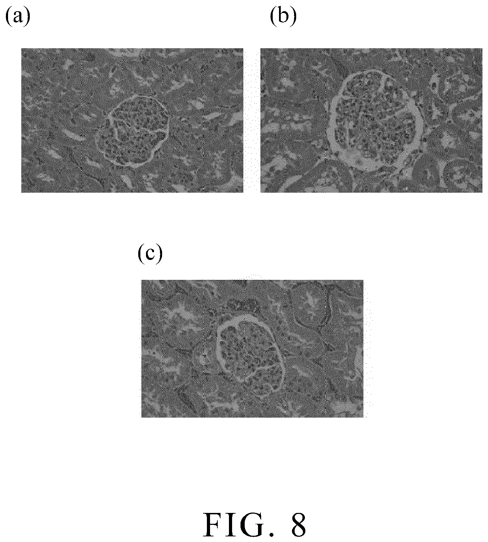

After staining the kidney slice of the experimental animal with H&E, it can be observed that the swelling and sclerosis of glomerulus of animal with 5/6 kidney resected is extremely serious; and after treatment with DL-P01-SI01 (J-TK), the situation is significantly improved, as shown in FIG. 8.

It can be known from the animal model obtained by 5/6 renal resection operation that, the alcohol extract of longan seeds exactly has the effects of decreasing excessively high blood and/or urine urinary protein, excessively high blood urea nitrogen, excessively high urine proteincreatinine ratio, excessively high blood and/or urine creatine kinase, excessively high blood and/or urine lactate dehydrogenase, excessively high blood and/or urine lactic acid and alleviating segmental sclerosis.

## Claims

1. An alcohol extract of longan seeds for use in the treatment of kidney tissue dysfunction,
wherein the alcohol extract of longan seeds comprises a fraction obtained by flash column chromatography; the column is 40g Silica; the flow rate is 18 mL/min; the mobile phase uses Solution A of ethyl acetate and Solution B of methanol; the gradient elution program is 100% Solution A and 0% Solution B at about 0 minute to about 30 minutes; 80% Solution A and 20% Solution B at about 31 minutes to about 45 minutes; 50% Solution A and 50% Solution B at about 46 minutes to about 65 minutes; 0% Solution A and 100% Solution B at about 66 minutes to about 85 minutes and at about 86 minutes to about 100 minutes.

2. The alcohol extract of longan seeds for use according to Claim 1, wherein the longan is DimocarpuslonganLour, Dimocarpuslongan or Dimocarpuslongan Fen Ke.

3. The alcohol extract of longan seeds for use according to Claim 1, wherein the alcohol is selected from the group consisting of methanol, ethanol, n-propanol, isopropanol, n-butanol, iso-butanol, sec-butanol, tert-butanol, and ethyl acetate or the solution thereof.

4. The alcohol extract of longan seeds for use according to Claim 1, wherein the alcohol is ethanol, ethyl acetate or n-butanol.

5. The alcohol extract of longan seeds for use according to Claim 1, wherein the alcohol extract of longan seeds comprises a fraction obtained by flash column chromatography with the gradient elution program of 100% Solution A and 0% Solution B at about 0 minute to about 30 minutes.

6. The alcohol extract of longan seeds for use according to Claim 1, wherein the alcohol extract of longan seeds is prepared according to a process comprising:
(a) providing longan seeds;
(b) cutting the longan seeds into small pieces; and
(c) extracting the small pieces in step (b) with the alcohol to obtain an extract.

7. The alcohol extract of longan seeds for use according to Claim 6, wherein the temperature for the extracting in step (c) is about 30°C to about 90°C.

8. The alcohol extract of longan seeds for use according to Claim 6, wherein the process further comprises a step (d) obtaining a liquid fraction from the extract.

9. The alcohol extract of longan seeds for use according to Claim 6, wherein step (c) further comprises:
(c1) extracting the small pieces in step (b) with an ethanol solution to obtain a rude extract;
(c2) lyophilizing the rude extract in step (c1); and
(c3) extracting a product of lyophilizing in step (c2) with ethyl acetate to obtain an ethyl acetate fraction and a water fraction.

10. The alcohol extract of longan seeds for use according to Claim 9, wherein step (c) further comprises (c4) extracting the water fraction with n-butanol to obtain an n-butanol sub-fraction and a water sub-fraction.

11. The alcohol extract of longan seeds for use according to Claim 9, wherein the ethyl acetate fraction comprises a sub-fraction obtained by flash column chromatography; the column is 40g Silica; the flow rate is 18 mL/min; the mobile phase uses Solution A of ethyl acetate and Solution B of methanol; the gradient elution program is 100% Solution A and 0% Solution B at about 0 minute to about 30 minutes; 80% Solution A and 20% Solution B at about 31 minutes to about 45 minutes; 50% Solution A and 50% Solution B at about 46 minutes to about 65 minutes; 0% Solution A and 100% Solution B at about 66 minutes to about 85 minutes and at about 86 minutes to about 100 minutes.

12. The alcohol extract of longan seeds for use according to Claim 1, wherein the kidney tissue dysfunction is caused by renal tissue necrosis, hypertension, immune injury, diabetes mellitus, systemic lupus erythematosus, aging, long-term drug abuse, family inheritance, high-salt diet, obesity, high cholesterol, smoking, alcohol or nephrectomy.

13. The alcohol extract of longan seeds for use according to Claim 1, wherein the kidney tissue dysfunction is selected from the group consisting of excessively high blood and/or urine urinary protein, excessively high blood urea nitrogen, excessively high urineprotein-creatinine ratio, excessively high blood and/or urine creatine kinase, excessively high blood and/or urine lactate dehydrogenase, excessively high blood and/or urine lactic acid and segmental sclerosis.

14. The alcohol extract of longan seeds for use according to Claim 1, wherein the kidney tissue dysfunction is nephritis.

## Patentansprüche

1. Alkoholextrakt aus Longansamen zur Verwendung bei der Behandlung von Nierengewebedysfunktion,
wobei der Alkoholextrakt von Longansamen eine durch Flash-Säulenchromatographie erhaltene Fraktion umfasst; wobei die Säule 40 g Kieselsäure ist; die Flussrate 18 mL/min beträgt; die mobile Phase Lösung A von Ethylacetat und Lösung B von Methanol verwendet; wobei das Gradienten-Elutionsprogramm 100% Lösung A und 0% Lösung B nach etwa 0 Minuten bis etwa 30 Minuten entspricht; 80% Lösung A und 20% Lösung B bei etwa 31 Minuten bis etwa 45 Minuten; 50% Lösung A und 50% Lösung B bei etwa 46 Minuten bis etwa 65 Minuten; 0% Lösung A und 100% Lösung B bei etwa 66 Minuten bis etwa 85 Minuten und bei etwa 86 Minuten bis etwa 100 Minuten entspricht.

2. Alkoholextrakt aus Longansamen zur Verwendung nach Anspruch 1, wobei das Longan DimocarpuslonganLour, Dimocarpuslongan oder Dimocarpuslongan Fen Ke ist.

3. Alkoholextrakt aus Longansamen zur Verwendung nach Anspruch 1, wobei der Alkohol ausgewählt ist aus der Gruppe bestehend aus Methanol, Ethanol, n-Propanol, Isopropanol, n-Butanol, Isobutanol, sec-Butanol, tert-Butanol und Ethylacetat oder der Lösung davon.

4. Alkoholextrakt aus Longansamen zur Verwendung nach Anspruch 1, wobei der Alkohol Ethanol, Ethylacetat oder n-Butanol ist.

5. Alkoholextrakt von Longansamen zur Verwendung nach Anspruch 1, wobei der Alkoholextrakt von Longansamen eine Fraktion umfasst, die durch Flash-Säulenchromatographie mit dem Gradientenelutions-Programm von 100% Lösung A und 0% Lösung B bei etwa 0 Minuten bis etwa 30 Minuten erhalten wurde.

6. Alkoholextrakt von Longansamen zur Verwendung nach Anspruch 1, wobei der Alkoholextrakt von Longansamen nach einem Verfahren hergestellt wird, das umfasst:
(a) Bereitstellung von Longansamen;
(b) Schneiden der Longansamen in kleine Stücke; und
(c) Extrahieren der kleinen Stücke in Schritt (b) mit dem Alkohol, um einen Extrakt zu erhalten.

7. Alkoholextrakt von Longansamen zur Verwendung nach Anspruch 6, wobei die Temperatur für die Extraktion in Schritt (c) etwa 30°C bis etwa 90°C beträgt.

8. Alkoholextrakt von Longansamen zur Verwendung nach Anspruch 6, wobei das Verfahren ferner einen Schritt (d) zum Erhalten einer flüssigen Fraktion aus dem Extrakt umfasst.

9. Alkoholextrakt von Longansamen zur Verwendung nach Anspruch 6, wobei Schritt (c) ferner umfasst:
(c1) Extrahieren der kleinen Stücke in Schritt (b) mit einer Ethanollösung, um einen groben Extrakt zu erhalten;
(c2) Lyophilisieren des unhöflichen Extrakts in Schritt (c1); und
(c3) Extrahieren eines Produkts der Lyophilisierung in Schritt (c2) mit Ethylacetat, um eine Ethylacetatfraktion und eine Wasserfraktion zu erhalten.

10. Alkoholextrakt von Longansamen zur Verwendung nach Anspruch 9, wobei Schritt (c) ferner (c4) das Extrahieren der Wasserfraktion mit n-Butanol umfasst, um eine n-Butanol-Subfraktion und eine Wassersubfraktion zu erhalten.

11. Alkoholextrakt aus Longansamen zur Verwendung nach Anspruch 9, wobei die Ethylacetatfraktion eine durch Flash-Säulenchromatographie erhaltene Subfraktion umfasst; wobei die Säule 40 g Kieselsäure ist; die Flussrate 18 mL/min beträgt; die mobile Phase Lösung A von Ethylacetat und Lösung B von Methanol verwendet; das Gradientenelutions-Programm 100% Lösung A und 0% Lösung B bei etwa 0 Minuten bis etwa 30 Minuten entspricht; 80% Lösung A und 20% Lösung B bei etwa 31 Minuten bis etwa 45 Minuten; 50% Lösung A und 50% Lösung B bei etwa 46 Minuten bis etwa 65 Minuten; 0% Lösung A und 100% Lösung B bei etwa 66 Minuten bis etwa 85 Minuten und bei etwa 86 Minuten bis etwa 100 Minuten entspricht.

12. Alkoholextrakt aus Longansamen zur Verwendung nach Anspruch 1, wobei die Nierengewebedysfunktion durch Nierengewebsnekrose, Bluthochdruck, Immunschwäche, Diabetes mellitus, systemischen Lupus erythematodes, Altern, langfristigen Drogenmissbrauch, Familienvererbung, salzreiche Ernährung, Übergewicht, hohen Cholesterinspiegel, Rauchen, Alkohol oder Nephrektomie verursacht wird.

13. Alkoholextrakt aus Longansamen zur Verwendung nach Anspruch 1, wobei die Nierengewebedysfunktion ausgewählt ist aus der Gruppe bestehend aus zu hohem Blut- und/oder Urinurinprotein, zu hohem Blut-Harnstoff-Stickstoff, zu hohem Urin-Protein-Creatinin-Verhältnis, zu hohem Blut- und/oder Urin-Creatinkinase, zu hohem Blut- und/oder Urin-Laktat-Dehydrogenase, zu hohem Blut- und/oder Urin-Milchsäuregehalt und Segmentalsklerose.

14. Alkoholextrakt aus Longansamen zur Verwendung nach Anspruch 1, wobei die Nierengewebedysfunktion eine Nephritis ist.

## Revendications

1. Un extrait alcoolique de graines de longane pour une utilisation dans le traitement du dysfonctionnement des tissus reinaux.
dans laquelle l'extrait alcoolique de graines de longane comprend une fraction obtenue par chromatographie sur colonne éclair; la colonne contenant 40 g de silice; le débit étant de 18 mL / min; la phase mobile utilisant une solution A d'acétate d'éthyle et une solution B de méthanol; le programme d'élution en gradient comprend 100% de solution A et 0% de solution B entre environ 0 minute jusqu'à environ 30 minutes; 80% de solution A et 20% de solution B à environ 31 minutes jusqu'à environ 45 minutes; 50% de solution A et 50% de solution B à environ 46 minutes et jusqu'à environ 65 minutes; 0% de solution A et 100% de solution B à environ 66 minutes et jusqu'à environ 85 minutes et à environ 86 minutes jusqu'à environ 100 minutes.

2. L'extrait alcoolique de graines de longane pour une utilisation selon la revendication 1, dans laquelle le longane est DimocarpuslonganLour, Dimocarpuslongan ou Dimocarpuslongan Fen Ke.

3. L'extrait alcoolique de graines de longane pour une utilisation selon la revendication 1, dans laquelle l'alcool est choisi dans le groupe constitué par le méthanol, l'éthanol, le n-propanol, l'isopropanol, le n-butanol, l'isobutanol, le sec-butanol, le tert-butanol et l'acétate d'éthyle ou leur solution.

4. L'extrait alcoolique de graines de longane pour une utilisation selon la revendication 1, dans laquelle l'alcool est l'éthanol, l'acétate d'éthyle ou le n-butanol.

5. L'extrait alcoolique de graines de longane pour une utilisation selon la revendication 1, dans laquelle l'extrait alcoolique de graines de longane comprend une fraction obtenue par chromatographie éclair sur colonne avec le programme d'élution en gradient de solution à 100% A et de solution B à 0% entre environ 0 minute et jusqu'à environ 30 minutes.

6. L'extrait alcoolique de graines de longane pour une utilisation selon la revendication 1, dans laquelle l'extrait d'alcool de graines de longane est préparé selon un procédé comprenant:
(a) la fourniture de graines de longane;
(b) couper les graines de longane en petits morceaux; et
(c) extraire les petits morceaux de l'étape (b) avec l'alcool pour obtenir un extrait.

7. L'extrait alcoolique de graines de longane pour une utilisation selon la revendication 6, dans laquelle la température pour l'extraction dans l'étape (c) est d'environ 30 ° C à environ 90 ° C.

8. L'extrait alcoolique de graines de longane pour une utilisation selon la revendication 6, dans laquelle le procédé comprend en outre une étape (d) consistant à obtenir une fraction liquide à partir de l'extrait.

9. L'extrait alcoolique de graines de longane pour une utilisation selon la revendication 6, dans laquelle l'étape (c) comprend en outre:
(c1) l'extraction des petits morceaux dans l'étape (b) avec une solution d'éthanol pour obtenir un extrait brut;
(c2) lyophiliser l'extrait brut à l'étape (c1); et
(c3) extraire un produit de lyophilisation dans l'étape (c2) avec de l'acétate d'éthyle pour obtenir une fraction d'acétate d'éthyle et une fraction d'eau.

10. L'extrait alcoolique de graines de longane pour une utilisation selon la revendication 9, dans laquelle l'étape (c) comprend en outre l'étape consistant à (c4) extraire la fraction d'eau avec du n-butanol pour obtenir une sous-fraction de n-butanol et une sous-fraction d'eau.

11. L'extrait alcoolique de graines de longane pour une utilisation selon la revendication 9, dans laquelle la fraction d'acétate d'éthyle comprend une sous-fraction obtenue par chromatographie sur colonne flash; la colonne contenant 40 g de silice; le débit étant de 18 mL / min; la phase mobile utilisant la solution A d'acétate d'éthyle et la solution B de méthanol; le programme d'élution en gradient comprenant 100% de solution A et 0% de solution B entre environ 0 minute et jusqu'à environ 30 minutes; 80% de solution A et 20% de solution B à environ 31 minutes à jusqu'à environ 45 minutes; 50% de solution A et 50% de solution B à environ 46 minutes et jusqu'à environ 65 minutes; 0% de solution A et 100% de solution B à environ 66 minutes et jusqu'à environ 85 minutes et à environ 86 minutes jusqu'à environ 100 minutes.

12. L'extrait alcoolique de graines de longane pour une utilisation selon la revendication 1, dans laquelle le dysfonctionnement des tissus rénaux est provoqué par une nécrose des tissus rénaux, une hypertension, une lésion immunitaire, un diabète sucré, un lupus érythémateux disséminé, un vieillissement, un abus de drogues à long terme, un héritage familial, un régime riche en sel, l'obésité, un taux de cholestérol élevé, tabagisme, alcool ou néphrectomie.

13. L'extrait alcoolique de graines de longane pour une utilisation selon la revendication 1, dans laquelle le dysfonctionnement des tissus rénaux est choisi dans le groupe comprenant une protéine urinaire dans le sang et/ou l'urine excessivement élevée, un taux trop élevé d'azote uréique dans le sang, un taux de protéine/créatinine excessivement élevé, une créatine kinase dans le sang et/ou l'urine excessivement élevée, une lactate déshydrogénase dans le sang et/ou les urines excessive, un acide lactique dans le sang et/ou les urines excessivement élevé et la sclérose en plaques.

14. L'extrait alcoolique de graines de longane pour une utilisation selon la revendication 1, dans laquelle le dysfonctionnement du tissu rénal est une néphrite.
